# FASCICULE DE BREVET EUROPEEN

(11) **EP 4 076 723 B1**
(45) Date de publication et mention de la délivrance du brevet: **11.02.2026**
(21) Numéro de dépôt: 20817035.7
(22) Date de dépôt: 08.12.2020
(51) Int. Cl.: B01J 19/24, C07C 2/08, C07C 2/38, B01J 12/00, B01J 10/00, B01J 19/00, C08F 10/00, C07C 2/10, C07C 2/32, C08F 2/06, C08F 210/16

(54) **REACTEUR GAZ/LIQUIDE D'OLIGOMERISATION A ZONES SUCCESSIVES DE DIAMETRE VARIABLE**
GASGEFÜLLTER FLÜSSIGER OLIGOMERISIERUNGSREAKTOR MIT AUFEINANDERFOLGENDEN ZONEN MIT VARIABLEN DURCHMESSERN
GAS/LIQUID OLIGOMERIZATION REACTOR HAVING SUCCESSIVE ZONES WITH VARIABLE DIAMETERS

(30) Priorité: 18.12.2019 FR 1914756
(43) Date de publication de la demande: 26.10.2022
(73) Titulaire: IFP Energies nouvelles, 92852 Rueil-Malmaison Cedex (FR)
(72) Inventeur: AUGIER, Frederic, 92852 RUEIL-MALMAISON CEDEX (FR); VONNER, Alexandre, 92852 RUEIL-MALMAISON CEDEX (FR); MAXIMIANO RAIMUNDO, Pedro, 92852 RUEIL-MALMAISON CEDEX (FR)
(74) Mandataire: IFP Energies nouvelles
(86) Numéro de dépôt international: PCT/EP2020/085019
(87) Numéro de publication internationale: WO 2021/122140

(56) Documents cités:
- EP-A1- 2 913 346
- WO-A1-2019/011806
- CN-A- 1 258 562
- DE-C1- 4 338 414

## Description

### DOMAINE TECHNIQUE

La présente invention concerne un réacteur gaz/liquide permettant l'oligomérisation comprenant une enceinte réactionnelle ayant des zones de diamètre décroissant du fond vers le sommet du réacteur. L'invention concerne également l'utilisation dudit réacteur gaz/liquide dans un procédé d'oligomérisation par catalyse homogène de l'éthylène en oléfines linéaires, et en particulier en but-1-ène, en hex-1-ène, et/ou en oct-1-ène.

### ART ANTERIEUR

L'invention concerne le domaine des réacteurs gaz/liquide encore appelés colonne à bulles, ainsi que leur mise en oeuvre dans un procédé d'oligomérisation de l'éthylène. Un inconvénient rencontré lors de la mise en oeuvre de tels réacteurs dans des procédés d'oligomérisation de l'éthylène est la gestion du ciel gazeux, correspondant à la partie supérieure du réacteur à l'état gazeux. Ledit ciel gazeux comprend les composés gazeux peu solubles dans la phase liquide, des composés partiellement solubles dans le liquide mais inertes, ainsi que de l'éthylène gazeux non dissous dans ledit liquide. Le passage de l'éthylène gazeux de la partie inférieure liquide de l'enceinte réactionnelle vers le ciel gazeux est un phénomène appelé perçage. Or le ciel gazeux est purgé afin d'éliminer lesdits composés gazeux. Lorsque la quantité d'éthylène gazeux présente dans le ciel gazeux est importante la purge du ciel gazeux entraine une perte en éthylène non négligeable ce qui nuit à la productivité et au coût du procédé d'oligomérisation. De plus, un phénomène de perçage important signifie que beaucoup d'éthylène gazeux n'a pas été dissous dans la phase liquide et donc n'a pas pu réagir ce qui nuit à la productivité et à la sélectivité du procédé d'oligomérisation.

Afin d'améliorer l'efficacité du procédé d'oligomérisation en terme de productivité et de coût, il est donc indispensable de limiter le phénomène de perçage de l'éthylène afin d'améliorer sa conversion dans ledit procédé tout en conservant un bonne sélectivité en alpha oléfines linéaires souhaitées.

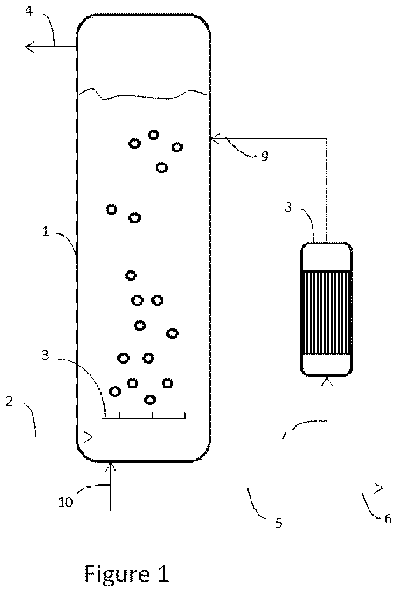

Les procédés de l'art antérieur mettant en oeuvre un réacteur gaz/liquide, tel qu'illustré à la figure 1, ne permettent pas de limiter la perte en éthylène gazeux et la purge du ciel gazeux entraine une sortie d'éthylène gazeux du réacteur néfaste pour le rendement et le coût du procédé.

La demanderesse a décrit des procédés dans les demandes WO2019/011806 et WO2019/011609 permettant d'augmenter la surface de contact entre la partie supérieure de la fraction liquide et le ciel gazeux par l'intermédiaire de moyen de dispersion ou de vortex afin de favoriser le passage de l'éthylène contenu dans le ciel gazeux vers la phase liquide au niveau de l'interface liquide/gaz. Ces procédés ne permettent pas de limiter le phénomène de perçage et ne sont pas suffisants lorsque la quantité d'éthylène dans le ciel gazeux est importante du fait d'un fort taux de perçage. DE 43 38 414 C1 divulgue un réacteur (2) d'oligomérisation gaz/liquide comprenant une enceinte réactionnelle de forme allongée le long d'un axe vertical et un moyen de purge d'une fraction gazeuse situé au sommet dudit réacteur, comprenant des plateaux dans ladite partie supérieure configurée pour recycler le solvant et les oléfines légères.

De plus lors de ces recherches, la demanderesse a constaté que dans un réacteur fonctionnant à débit constant d'éthylène gazeux injecté, la quantité d'éthylène dissous et donc le taux de perçage est dépendant des dimensions des réacteurs mettant en oeuvre le procédé et notamment de la hauteur de la phase liquide. En effet, plus la hauteur est faible plus le temps durant lequel l'éthylène gazeux parcourt la phase liquide pour se dissoudre est faible et plus le taux de perçage est élevé.

La demanderesse a découvert qu'il est possible d'améliorer la conversion d'oléfine(s), tout en conservant une sélectivité élevée en oléfine(s) linéaire(s) recherchée(s), et notamment en alpha-oléfine(s), en limitant les phénomènes de perçages au moyen d'un réacteur gaz/liquide ayant des zones successives de diamètre décroissant du fond vers le sommet du réacteur.

Avantageusement, un réacteur selon la présente invention permet d'augmenter la hauteur du réacteur et donc la hauteur de la phase liquide sans modifier le volume du réacteur ni de la phase liquide mis en oeuvre dans une réaction d'oligomérisation, ce qui a pour effet d'améliorer la dissolution de l'éthylène gazeux et donc de limiter le phénomène de perçage pour un volume de phase liquide donné.

L'invention permet donc, pour un volume de phase liquide donné, d'augmenter la hauteur de la phase liquide par rapport à un réacteur de diamètre constant.

L'invention porte également sur un procédé d'oligomérisation d'oléfines et en particulier de l'éthylène mettant en oeuvre le réacteur à zones successives de diamètre décroissant selon l'invention.

### DESCRIPTION SOMMAIRE DE L'INVENTION

La présente invention concerne donc un réacteur gaz/liquide à zones consécutives de diamètres décroissants comprenant :
- une enceinte réactionnelle 1, de forme allongée le long de l'axe vertical,
- un moyen d'introduction d'éthylène gazeux 2, situé dans le fond de l'enceinte réactionnelle,
- un moyen de soutirage 5 d'un effluent liquide réactionnel situé dans le fond de l'enceinte réactionnelle,
- un moyen de purge 4 d'une fraction gazeuse situé au sommet dudit réacteur,
   dans lequel
- ladite enceinte est composée de n zones consécutives ayant un diamètre Dn décroissant dans le sens de la zone de fond vers la zone du sommet de ladite enceinte,
- le rapport (Dn/Dn-1) du diamètre de la zone supérieure, noté Dn, sur le diamètre de la zone inférieure adjacente, noté Dn-1, est inférieur ou égale à 0,9
- pour une zone donnée le rapport du volume noté Vn, sur le volume total de l'enceinte réactionnelle, noté Vtot, est compris entre 0,2 et 0,8.
- les n zones consécutives sont disposées en série selon l'axe vertical du réacteur de manière à définir des zones dans l'enceinte réactionnelle ayant des diamètres décroissants du fond vers le sommet et ainsi d'augmenter la hauteur d'une phase liquide pouvant être contenu dans ladite enceinte réactionnelle par rapport à la hauteur d'un réacteur de diamètre constant.

Dans un mode de réalisation préféré, le nombre n de zones est compris entre 2 et 5.

Dans un mode de réalisation préféré, le rapport (Dn/Dn-1) du diamètre d'une zone supérieure n sur le diamètre de la zone inférieure adjacente n-1 est compris entre 0,1 et 0,9.

Dans un mode de réalisation préféré, le rapport (Hn/Hn-1) de la hauteur d'une zone supérieure n, noté Hn, sur la hauteur de la zone inférieure adjacente n-1, noté Hn-1, est compris entre 0,2 et 3,0, de préférence entre 0,3 et 2,5.

Dans un mode de réalisation préféré, pour une zone donnée le rapport du volume noté Vn, sur le volume total, noté Vtot, (noté Vn/Vtot) de l'enceinte réactionnelle correspondant à la somme des n zones est compris entre 0,2 et 0,8, de préférence entre 0,25 et 0,75.

Dans un mode de réalisation préféré, les n zones composant ladite enceinte sont formées par l'assemblage de cylindres de diamètre décroissant.

Dans un mode de réalisation préféré, les n zones composant ladite enceinte sont formées par des internes positionnés à l'intérieur de l'enceinte réactionnelle de manière à diminuer son diamètre sur une zone donnée.

Dans un mode de réalisation préféré, le réacteur comprend en outre une boucle de recirculation comprenant un moyen de soutirage sur la partie inférieure de l'enceinte réactionnelle, de préférence au fond, de manière à soutirer une fraction liquide vers un ou plusieurs échangeur(s) thermique(s) apte au refroidissement de ladite fraction liquide, et un moyen d'introduction de ladite fraction refroidie dans la partie supérieure de l'enceinte réactionnelle.

Dans un mode de réalisation préféré, le réacteur en outre un moyen de soutirage d'une fraction gazeuse au niveau du ciel gazeux de l'enceinte réactionnelle et un moyen d'introduction de ladite fraction gazeuse soutirée dans la phase liquide dans la partie inférieure de l'enceinte réactionnelle.

Un autre objet de la présente invention concerne un procédé d'oligomérisation d'éthylène gazeux mettant en œuvre le réacteur selon l'un quelconque des modes de réalisation précédents.

Dans un mode de réalisation préféré, le procédé est mis en œuvre à une pression comprise entre 0,1 et 10,0 MPa, à une température comprise entre 30 et 200°C comprenant les étapes suivantes :
- une étape a) d'introduction d'un système catalytique d'oligomérisation comprenant un catalyseur métallique et un agent activateur, dans une enceinte réactionnelle,
- une étape b) de mise en contact dudit système catalytique avec de l'éthylène gazeux par l'introduction dudit éthylène gazeux dans la zone inférieure de l'enceinte réactionnelle,
- une étape c) de soutirage d'une fraction liquide,
- une étape d) de refroidissement de la fraction soutirée à l'étape c) par le passage de ladite fraction dans un échangeur thermique,
- une étape e) d'introduction de la fraction refroidie à l'étape d) dans la partie supérieure de la zone inférieure de l'enceinte réactionnelle.

Dans un mode de réalisation préféré, le procédé comprend en outre une étape de recyclage d'une fraction gazeuse soutirée de la zone supérieure de l'enceinte réactionnelle et introduite au niveau de la partie inférieure de l'enceinte réactionnelle dans la phase liquide.

### DEFINITIONS & ABREVIATIONS

Dans l'ensemble de la description les termes ou abréviations ci-après ont le sens suivant.

Le terme « oligomérisation » désigne toute réaction d'addition d'une première oléfine sur une seconde oléfine, identique ou différente de la première et comprend la dimérisation, la trimérisation et la tétramérisation. L'oléfine ainsi obtenue de type CₙH₂ₙ où n est égal ou supérieur à 4.

Le terme « oléfine » désigne aussi bien une oléfine qu'un mélange d'oléfines.

Le terme « alpha-oléfine » désigne une oléfine, sur laquelle la double liaison est située en position terminale de la chaine alkyle.

Le terme « hétéroatome » est un atome différent du carbone et de l'hydrogène. Un hétéroatome peut être choisi parmi l'oxygène, le soufre, l'azote, le phosphore, le silicium et les halogénures tels que le fluor, le chlore, le brome ou l'iode.

Le terme « hydrocarbure » est un composé organique constitué exclusivement d'atomes de carbone (C) et d'hydrogène (H) de formule brute CₘHₚ, avec m et p des entiers naturels.

Le terme « système catalytique » désigne un mélange d'au moins un précurseur métallique, d'au moins un agent activateur, éventuellement d'au moins un additif et éventuellement d'au moins un solvant.

La terme « alkyle » est une chaine hydrocarbonée comprenant entre 1 et 20 atomes de carbone, préférentiellement de 2 à 15 atomes de carbone et encore plus préférentiellement de 2 à 8 atomes de carbone, noté alkyle en C₁-C₂₀, saturée ou non, linéaire ou ramifiée, non cyclique, cyclique ou polycyclique. Par exemple, on entend par alkyle en C₁-C₆, un alkyle choisi parmi les groupements méthyle, éthyle, propyle, butyle, pentyle, cyclopentyle, hexyle et cyclohexyle.

Le terme « aryle » est un groupement aromatique, mono ou polycyclique, fusionné ou non, comprenant entre 6 et 30 atomes de carbone, noté aryle en C₆-C₃₀.

Le terme « alcoxy » est un radical monovalent constitué d'un groupement alkyl lié à un atome d'oxygène tel que le groupement C₄H₉O-.

Le terme « aryloxy » est un radical monovalent constitué d'un groupement aryle lié à un atome d'oxygène tel que le groupement C₆H₅O-.

On entend par phase liquide, le mélange de l'ensemble des composés qui se trouvent à un état physique liquide dans les conditions de température et de pression de l'enceinte réactionnelle, ladite phase pouvant comprendre des composés gazeux tel que l'éthylène gazeux.

On entend par partie inférieure, la partie de l'enceinte se situant au niveau de la phase liquide, et ladite phase pouvant comprendre de l'éthylène gazeux, les produits de la réaction tels que l'alpha oléfine linéaire souhaitée (i.e butène-1, hexène-1, octène-1), un ou plusieurs solvants et un système catalytique.

On entend par ciel gazeux, la partie supérieure de l'enceinte à l'état gazeux se situant au sommet de l'enceinte réactionnelle, c'est-à-dire directement au-dessus de la phase liquide et constitué d'un mélange de composés qui se trouvent à l'état physique gaz lors de la mise en œuvre d'un réacteur dans un procédé d'oligomérisation.

On entend par partie inférieure latérale de l'enceinte réactionnelle une partie de l'enveloppe de l'enceinte réactionnelle du réacteur située en partie basse et sur le côté.

On entend par gaz incondensable une espèce sous forme physique gaz qui ne se dissout que partiellement dans le liquide aux conditions de température et de pression de l'enceinte réactionnelle, et qui peut, dans certaines conditions, s'accumuler dans le ciel du réacteur (exemple ici : l'éthane).

On entend par t/h, la valeur d'un débit exprimée en tonne par heure et par kg/s, la valeur d'un débit en kilogramme par seconde.

On désigne par les termes réacteur ou dispositif, l'ensemble des moyens permettant la mise en œuvre du procédé d'oligomérisation selon l'invention, tel que notamment l'enceinte réactionnelle et la boucle de recirculation.

On entend par fond de l'enceinte réactionnelle le quart inférieur de l'enceinte réactionnelle.

On entend par sommet de l'enceinte réactionnelle le quart supérieur de l'enceinte réactionnelle.

On entend par zone de fond, la première zone selon l'invention se situant dans la partie inférieure de l'enceinte réactionnelle au niveau du fond de ladite enceinte.

On entend par zone du sommet, la dernière zone selon l'invention se situant dans la partie supérieure de l'enceinte réactionnelle au niveau du sommet de ladite enceinte.

On entend par éthylène gazeux frais, l'éthylène extérieur au procédé introduit à l'étape b) par le moyen du procédé selon l'invention.

On entend par phénomène de perçage, le passage de l'éthylène gazeux non dissous dans la phase liquide vers le ciel gazeux.

On entend par taux de saturation, le pourcentage d'éthylène dissous dans la phase liquide par rapport à la quantité maximale d'éthylène qui pourrait être dissoute dans ladite phase liquide, défini par l'équilibre thermodynamique entre la pression partielle d'éthylène gazeuse et ladite phase liquide. Le taux de saturation peut être mesuré par chromatographie en phase gazeuse.

### DESCRIPTION DES FIGURES

La figure 1 illustre un réacteur gaz/liquide selon l'art antérieur. Ce dispositif est constitué d'une enceinte réactionnelle 1 comprenant une partie inférieure comprenant une phase liquide, une partie supérieure comprenant un ciel gazeux, et un moyen d'introduction de l'éthylène gazeux 2 par l'intermédiaire d'un distributeur gazeux 3 dans la phase liquide. La partie supérieure comprend un moyen de purge 4. Dans le fond de l'enceinte réactionnelle 1 se situe une conduite pour le soutirage d'une fraction liquide 5. Ladite fraction 5 est divisée en deux flux, un premier flux principal 7 envoyé vers un échangeur à chaleur 8 puis introduit par l'intermédiaire d'une conduite 9 dans la phase liquide et un second flux 6 correspondant à l'effluent envoyé vers une étape ultérieure. La conduite 10 dans le fond de l'enceinte réactionnelle permet l'introduction du système catalytique.

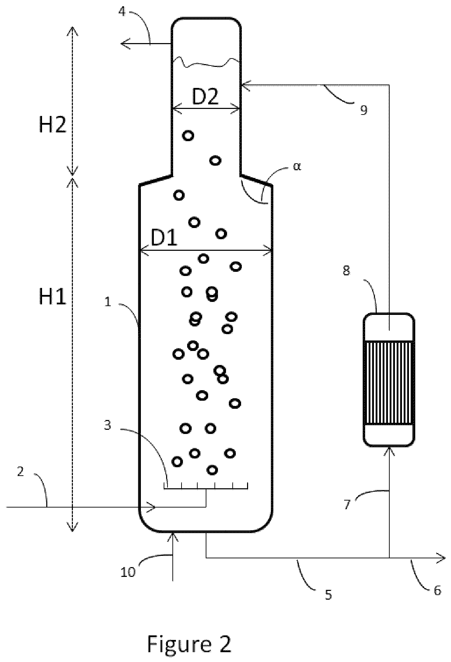

La figure 2 illustre un réacteur gaz/liquide à zones consécutives de diamètre décroissant selon l'invention. Ledit réacteur diffère du réacteur de la figure 1 en ce qu'il comprend deux zones de diamètre différent. La zone 1 située en fond de l'enceinte réactionnelle à un diamètre supérieur à la zone située au sommet de ladite enceinte. La première zone de fond est caractérisée par son diamètre noté D1 et sa hauteur H1, ces deux paramètres définissant le volume, noté V1, de ladite zone. De manière similaire, la deuxième zone située au sommet est caractérisée par sa hauteur notée H2 et son diamètre noté D2, inférieur à D1, définissant le volume, V2, de la deuxième zone. Dans ce mode de réalisation, les deux zones composant l'enceinte réactionnelle 1 sont formées de cylindres de diamètre décroissant.

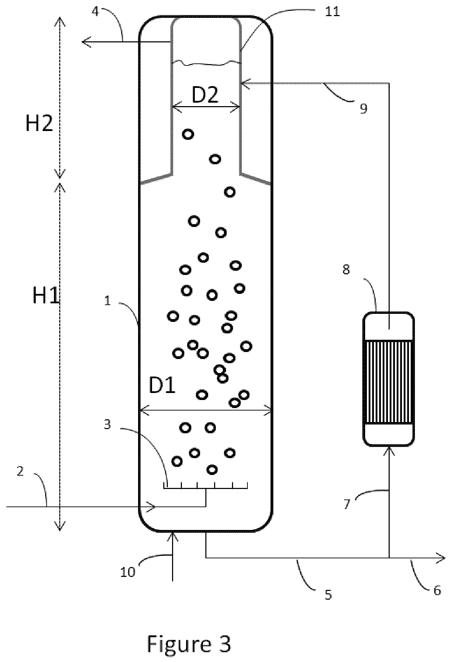

La figure 3 illustre un autre mode de réalisation qui diffère de celui de la figure 2 en ce que la deuxième zone située au sommet de l'enceinte réactionnelle 1 est délimitée par un interne 11 positionné à l'intérieur de l'enceinte réactionnelle 1.

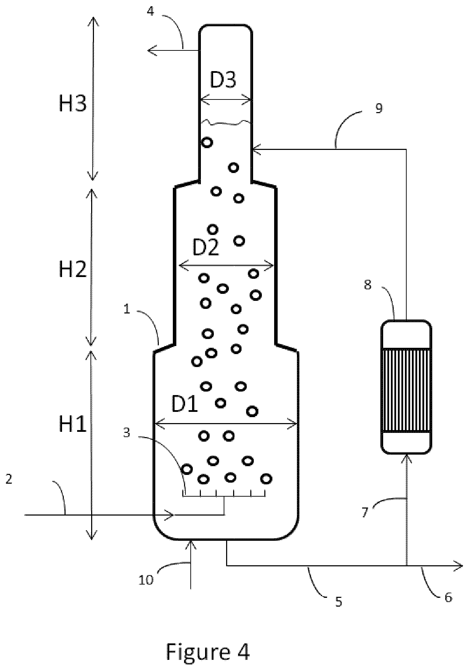

La figure 4 illustre un autre mode de réalisation qui diffère de celui de la figure 2 en ce que l'enceinte réactionnelle 1 comprend trois zones consécutives de diamètre décroissant.

Les figures 2, 3 et 4 illustrent schématiquement des modes de réalisation particuliers de l'objet de la présente invention sans en limiter la portée.

### DESCRIPTION DETAILLEE DE L'INVENTION

Il est précisé que, dans toute cette description, l'expression « compris(e) entre ... et ... » doit s'entendre comme incluant les bornes citées.

Dans le sens de la présente invention, les différents modes de réalisation présentés peuvent être utilisés seuls ou en combinaison les uns avec les autres, sans limitation de combinaison lorsque c'est techniquement réalisable.

Dans le sens de la présente invention, les différentes plages de paramètres pour une étape donnée tels que les plages de pression et les plages de température peuvent être utilisées seul ou en combinaison. Par exemple, dans le sens de la présente invention, une plage de valeur préférée de pression peut être combinée avec une plage de valeur de température plus préférée.

La présente invention concerne donc un réacteur d'oligomérisation gaz/liquide à zones consécutives de diamètre décroissant comprenant :
- une enceinte réactionnelle 1, de forme allongée le long de l'axe vertical,
- un moyen d'introduction d'éthylène gazeux 2, situé dans le fond de l'enceinte réactionnelle,
- un moyen de soutirage 5 d'un effluent liquide réactionnel situé dans le fond de l'enceinte réactionnelle,
- un moyen de purge 4 d'une fraction gazeuse situé au sommet dudit réacteur,
   dans lequel
- ladite enceinte est composée de n zones consécutives ayant un diamètre Dn décroissant dans le sens de la zone de fond vers la zone du sommet de ladite enceinte,
- le rapport (Dn/Dn-1) du diamètre de la zone supérieure, noté Dn, sur le diamètre de la zone inférieure adjacente, noté Dn-1, est inférieur ou égale à 0,9,
- pour une zone donnée le rapport du volume noté Vn, sur le volume total de l'enceinte réactionnelle, noté Vtot, est compris entre 0,2 et 0,8.

Avantageusement, un réacteur selon la présente invention permet d'augmenter la hauteur du réacteur et donc de la phase liquide sans modifier le volume de liquide mis en œuvre dans une réaction d'oligomérisation ce qui a pour effet d'améliorer la dissolution de l'éthylène gazeux et donc de limiter le phénomène de perçage pour un volume de phase liquide donné.

### Une enceinte réactionnelle

L'enceinte réactionnelle 1 selon l'invention comprend donc
- n zones consécutives ayant un diamètre Dn décroissant dans le sens de la zone de fond vers la zone du sommet de ladite enceinte,
- le rapport (Dn/Dn-1) du diamètre de la zone supérieure, noté Dn, sur le diamètre de la zone inférieure adjacente, noté Dn-1, est inférieur ou égale à 0,9,
- pour une zone donnée le rapport du volume, noté Vn, sur le volume total de l'enceinte réactionnelle, noté Vtot, est compris entre 0,2 et 0,8.
- n zones consécutives selon l'invention sont disposées en série selon l'axe vertical du réacteur de manière à définir des zones dans l'enceinte réactionnelle ayant des diamètres décroissants du fond vers le sommet et ainsi d'augmenter la hauteur de la phase liquide pouvant être contenue dans l'enceinte réactionnelle par rapport à la hauteur d'un réacteur de diamètre constant, et donc le temps durant lequel l'éthylène est présent dans la phase liquide de manière à améliorer sa dissolution.

Avantageusement, pour un volume d'enceinte réactionnelle donné et donc un volume de liquide donné, les n zones consécutives de diamètre décroissant dans ladite enceinte réactionnelle permettent d'augmenter la hauteur du liquide pouvant être contenue dans ladite enceinte et ainsi le temps de séjour de l'éthylène gazeux introduit dans ladite phase liquide. Ainsi, la présente invention permet d'augmenter la quantité d'éthylène dissous dans la phase liquide et donc de limiter le phénomène de perçage.

De préférence, l'enceinte réactionnelle comprend un nombre n de zones compris entre 2 et 5, de préférence entre 2 et 4 et de manière préférée n est égal à 2, 3, 4 ou 5.

Le rapport (Dn/Dn-1) du diamètre d'une zone supérieure n, noté Dn, sur le diamètre de la zone inférieure adjacente n-1, noté Dn-1, est inférieur ou égal à 0,9. De préférence le rapport Dn/Dn-1 est compris entre 0,1 et 0,9, de préférence entre 0,15 et 0,85, de préférence entre 0,2 et 0,8 et de manière préférée entre 0,25 et 0,75 et de manière très préférée entre 0,3 et 0,7.

Les n zones composant l'enceinte réactionnelle ont une hauteur totale, notée Htot, dont la somme est égale à la hauteur totale de l'enceinte réactionnelle.

Avantageusement, le rapport (Hn/Hn-1) de la hauteur d'une zone supérieure n, notée Hn, sur la hauteur de la zone inférieure adjacente n-1, notée Hn-1, est compris entre 0,2 et 3,0, de préférence entre 0,3 et 2,5, de préférence entre 0,4 et 2,0, de préférence entre 0,5 et 1,5 et de manière préférée entre 0,6 et 1,0
De préférence, pour une zone donnée le rapport du volume noté Vn, sur le volume total, noté Vtot, (noté Vn/Vtot) de l'enceinte réactionnelle correspondant à la somme des n zones est compris entre 0,2 et 0,8. De préférence ledit rapport (Vn/Vtot) est compris entre 0,25 et 0,75, de préférence entre 0,3 et 0,7 et de manière préférée entre 0,35 et 0,65.

De préférence, l'enceinte réactionnelle est de forme cylindrique et présente un rapport hauteur totale de l'enceinte sur le diamètre de la zone de fond de ladite enceinte (noté Htot/D1) compris entre 1 et 17, de préférence entre 1 et 8, et de manière préférée entre 2 et 7.

Dans un premier mode de réalisation particulier représenté à la figure 2, les n zones composant ladite enceinte sont formées de cylindres de diamètre décroissant. Lesdits cylindres sont reliés entre eux par l'intermédiaire de parois perpendiculaires à l'axe vertical ou présentant un angle α entre 90 et 160° avec l'axe vertical, représenté sur la figure 2, de manière à faciliter et surtout ne pas bloquer l'ascension des bulles d'éthylène gazeux dans la phase liquide. De préférence, ledit angle est compris entre 95 et 145°, et de manière préférée entre 100 et 130°.

Dans un second mode de réalisation particulier représenté à la figure 3, les n zones composant ladite enceinte sont formées par des internes positionnés à l'intérieur de l'enceinte réactionnelle de manière à diminuer son diamètre sur une zone donnée. Lesdits internes peuvent être par exemple des parois métalliques pleines.

Avantageusement, quel que soit le mode de réalisation la solidarisation de l'enceinte réactionnelle est réalisée par fixation des cylindres et/ou des internes, par exemple par soudage, par collage, par vissage, par boulonnage seul ou en combinaison, ou tout autre moyen analogue. De préférence, la fixation est mise en œuvre par soudage.

De préférence, l'enceinte réactionnelle comprend également un moyen de purge des gaz incondensables au niveau du ciel gazeux.

De préférence, l'enceinte réactionnelle comprend également un capteur de pression, permettant de contrôler la pression au sein de l'enceinte réactionnelle, et de préférence de maintenir constante la pression. De préférence, en cas de diminution de la pression, ladite pression est maintenue constante par l'introduction d'éthylène gazeux dans l'enceinte réactionnelle.

### Un moyen d'introduction de l'éthylène gazeux

Selon l'invention, l'enceinte réactionnelle comprend un moyen d'introduction de l'éthylène gazeux situé dans le fond de ladite enceinte, plus particulièrement dans la partie inférieure latérale.

De préférence le moyen d'introduction de l'éthylène est choisi parmi une conduite, un réseau de conduites, un distributeur multitubulaire, une plaque perforée ou tout autre moyen connu de l'Homme du métier.

Dans un mode de réalisation particulier, le moyen d'introduction de l'éthylène est situé dans la boucle de recirculation.

De préférence, un distributeur gazeux, qui est un dispositif permettant de disperser la phase gaz de manière uniforme sur toute la section liquide, est positionné à l'extrémité du moyen d'introduction au sein de l'enceinte réactionnelle. Ledit dispositif comprend un réseau de conduites perforées, dont le diamètre des orifices est compris entre 1,0 et 12,0 mm, de préférence entre 3,0 et 10,0 mm, pour former des bulles d'éthylène dans le liquide de dimension millimétrique.

### Un moyen optionnel d'introduction du système catalytique

Selon l'invention, l'enceinte réactionnelle comprend un moyen d'introduction du système catalytique.

De préférence, le moyen d'introduction est situé en fond de ladite enceinte.

Selon une variante de réalisation, l'introduction du système catalytique est réalisée dans la boucle de recirculation.

Le moyen d'introduction du système catalytique est choisi parmi tout moyen connu de l'Homme du métier et de préférence est une conduite.

Dans le mode de réalisation où le système catalytique est mis en œuvre en présence d'un solvant ou d'un mélange de solvants, ledit solvant ou ledit mélange de solvants est introduit par un moyen d'introduction situé dans le fond de l'enceinte réactionnelle ou encore dans la boucle de recirculation.

### Une boucle optionnelle de recirculation

Avantageusement, l'homogénéité de la phase liquide, ainsi que la régulation de la température au sein de l'enceinte réactionnelle du réacteur selon l'invention peuvent être réalisées par l'utilisation d'une boucle de recirculation comprenant un moyen de soutirage sur la partie inférieure de l'enceinte réactionnelle, de préférence au fond, de manière à réaliser le soutirage d'une fraction liquide vers un ou plusieurs échangeur(s) thermique(s) permettant le refroidissement de ladite fraction liquide, et un moyen d'introduction de ladite fraction liquide refroidie dans la partie supérieure de l'enceinte réactionnelle, de préférence au niveau de la phase liquide.

La boucle de recirculation permet une bonne homogénéisation des concentrations et de contrôler la température dans la phase liquide au sein de l'enceinte réactionnelle.

Avantageusement, la mise en œuvre d'une boucle de recirculation permet d'induire un sens de circulation de la phase liquide dans l'enceinte réactionnelle de la partie supérieure vers la partie inférieure de ladite enceinte ce qui permet d'augmenter le temps de séjour de l'éthylène gazeux en ralentissant sa montée dans ladite phase liquide et donc de limiter encore le phénomène de perçage.

La boucle de recirculation peut avantageusement être mise en œuvre par tout moyen nécessaire et connu de l'Homme du métier, tel que, une pompe pour le soutirage de la fraction liquide, un moyen apte à réguler le débit de la fraction liquide soutirée, ou encore une conduite de purge d'au moins une partie de la fraction liquide.

De préférence le moyen de soutirage et le moyen de d'introduction de la fraction liquide de l'enceinte réactionnelle sont une conduite.

Le ou les échangeur(s) thermique(s) apte(s) à refroidir la fraction liquide est (sont) choisi(s) parmi tout moyen connu de l'Homme du métier.

### Une boucle optionnelle de recycle du ciel gazeux

Avantageusement, le réacteur gaz/liquide d'oligomérisation à zones consécutives de diamètre variable comprend en outre une boucle de recycle du ciel gazeux dans la partie inférieure de l'enceinte réactionnelle au niveau de la phase liquide. Ladite boucle comprenant un moyen de soutirage d'une fraction gazeuse au niveau du ciel gazeux de l'enceinte réactionnelle et un moyen d'introduction de ladite fraction gazeuse soutirée dans la phase liquide dans la partie inférieure de l'enceinte réactionnelle.

La boucle de recycle permet avantageusement de compenser le phénomène de perçage et d'éviter l'augmentant de la pression dans l'enceinte réactionnelle, tout en maintenant la saturation en éthylène dissous dans la phase liquide à une valeur souhaitée.

Un autre avantage de la boucle de recycle est d'améliorer la productivité volumique du dispositif et donc de diminuer les coûts. Dans un mode de réalisation préféré, la boucle de recycle comprend en outre un compresseur.

Dans un mode de réalisation, l'introduction de la fraction gazeuse soutirée est réalisée par l'intermédiaire du moyen d'introduction de l'éthylène gazeux.

Dans un autre mode de réalisation, l'introduction de la fraction gazeuse soutirée est réalisée par l'intermédiaire d'un distributeur gazeux qui est un dispositif permettant de disperser la phase gaz de manière uniforme sur toute la section liquide, et positionné à l'extrémité du moyen d'introduction au sein de l'enceinte réactionnelle. Ledit dispositif comprend un réseau de conduites perforées, dont le diamètre des orifices est compris entre 1,0 et 12,0 mm, de préférence entre 3,0 et 10,0 mm, pour former des bulles d'éthylène dans le liquide de dimension millimétrique.

De préférence le moyen d'introduction de la fraction gazeuse soutirée est choisi parmi une conduite, un réseau de conduites, un distributeur multitubulaire, une plaque perforée ou tout autre moyen connu de l'Homme du métier.

### Procédé d'oligomérisation

Un autre objet de la présente invention couvre un procédé d'oligomérisation mettant en oeuvre le réacteur à zones de diamètre variable selon l'invention tel que décrit précédemment.

De préférence, dans un réacteur gaz/liquide le débit d'éthylène gazeux introduit à l'étape b) telle que définie ci-après, est asservi à la pression dans l'enceinte réactionnelle. Ainsi, en cas d'augmentation de la pression dans le réacteur du fait d'un fort taux de perçage de l'éthylène dans le ciel gazeux, le débit d'éthylène gazeux introduit à l'étape b), telle que définie ci-après, diminue ce qui entraine une diminution de la quantité d'éthylène dissous dans la phase liquide, donc de la saturation en éthylène. Ladite diminution est préjudiciable pour la conversion de l'éthylène et s'accompagne d'une diminution de la productivité du réacteur, et éventuellement de sa sélectivité.

Avantageusement la mise en œuvre du réacteur à zones de diamètre variable selon l'invention dans un procédé d'oligomérisation, de préférence par catalyse homogène, permet d'avoir un taux de saturation en éthylène dissous dans la phase liquide supérieur à 70,0 %, de préférence entre 70,0 et 100 %, de préférence entre 80,0 et 100 %, de manière préférée compris entre 80,0 et 99,0 %, de préférence entre 85,0 et 99,0 % et de manière encore plus préférée entre 90,0 et 98,0 %.

Le taux de saturation en éthylène dissous peut être mesuré par toute méthode connue de l'Homme du métier et par exemple par l'analyse chromatographique en phase gaz (couramment appelée GC) d'une fraction de la phase liquide soutirée de l'enceinte réactionnelle.

Le procédé mettant en œuvre le réacteur à zones de diamètre variable selon l'invention permet l'obtention d'oléfines linéaires et particulièrement d'alpha-oléfines linéaires par la mise en contact d'oléfine(s) et d'un système catalytique, éventuellement en présence d'un additif et/ou d'un solvant, et par la mise en œuvre dudit réacteur gaz/liquide à zones de diamètre variable.

Tous les systèmes catalytiques connus de l'Homme du métier et aptes à être mis en œuvre dans les procédés de dimérisation, de trimérisation, de tétramérisation et plus généralement dans les procédés d'oligomérisation selon l'invention, font partie du domaine de l'invention. Lesdits systèmes catalytiques ainsi que leurs mises en œuvres sont notamment décrits dans les demandes FR2984311, FR2552079, FR3019064, FR3023183, FR3042989 ou encore dans la demande FR3045414.

De préférence, les systèmes catalytiques comprennent, de préférence sont constitués de :
- un précurseur métallique de préférence à base de nickel, de titane, ou de chrome,
- un agent activateur,
- optionnellement un additif, et
- optionnellement un solvant.

### Le précurseur métallique

Le précurseur métallique utilisé dans le système catalytique est choisi parmi les composés à base de nickel, de titane ou de chrome.

Dans un mode de réalisation, le précurseur métallique est à base de nickel et préférentiellement comprend du nickel de degré d'oxydation (+II). De préférence, le précurseur de nickel est choisi parmi les carboxylates de nickel(II) tel que par exemple le 2-éthylhexanoate de nickel, les phénates de nickel(II), les naphténates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydratée ou non, pris seul ou en mélange.

Dans un second mode de réalisation, le précurseur métallique est à base de titane et préférentiellement comprend un composé aryloxy ou alcoxy du titane.

Le composé alcoxy du titane répond avantageusement à la formule générale [Ti(OR)₄] dans laquelle R est un radical alkyle linéaire ou ramifié. Parmi les radicaux alcoxy préférés, on peut citer à titre d'exemple non limitatifs : le tétraéthoxy, le tétraisopropoxy, le tétra-n-butoxy et le tétra-2-éthyl-hexyloxy.

Le composé aryloxy du titane répond avantageusement à la formule générale [Ti(OR')_{4]} dans laquelle R' est un radical aryle substitué ou non par des groupements alkyle ou aryle. Le radical R' peut comporter des substituants à base d'hétéroatome. Les radicaux aryloxy préférés sont choisis parmi le phénoxy, le 2-méthylphénoxy, le 2,6-diméthylphénoxy, le 2,4,6-triméthylphénoxy, le 4-méthylphénoxy, le 2-phénylphénoxy, le 2,6-diphénylphénoxy, le 2,4,6-triphénylphénoxy, le 4-phénylphénoxy, le 2-tert-butyl-6-phénylphénoxy, le 2,4-ditertbutyl-6-phénylphénoxy, le 2,6-diisopropylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy, le radical biphénoxy, le binaphtoxy, le 1,8-naphtalène-dioxy.

Selon un troisième mode de réalisation, le précurseur métallique est à base de chrome et préférentiellement comprend un sel de chrome (II), un sel de chrome (III), ou un sel de degré d'oxydation différent pouvant comporter un ou plusieurs anions identiques ou différents, tels que par exemple des halogénures, des carboxylates, des acétylacétonates, des anions alcoxy ou aryloxy. De préférence, le précurseur à base de chrome est choisi parmi CrCl₃, CrCl₃(tétrahydrofurane)₃, Cr(acétylacétonate)₃, Cr(naphténate)₃, Cr(2-éthylhexanoate)₃, Cr(acétate)₃.

La concentration en nickel, en titane ou en chrome, est comprise entre 0,01 et 300,0 ppm en masse de métal atomique par rapport à la masse réactionnelle, de préférence entre 0,02 et 100,0 ppm, préférentiellement entre 0,03 et 50,0 ppm, plus préférentiellement entre 0,5 et 20,0 ppm et encore plus préférentiellement entre 2,0 et 50,0 ppm en masse de métal atomique par rapport à la masse réactionnelle.

### L'agent activateur

Quel que soit le précurseur métallique, le système catalytique comprend en outre un ou plusieurs agents activateurs choisis parmi les composés à base d'aluminium tels que, le dichlorure de méthylaluminium (MeAlCl₂), le dichloroéthylaluminium (EtAlCl₂), le sesquichlorure d'éthylaluminium (Et₃Al₂Cl₃), le chlorodiéthylaluminium (Et₂AlCl), le chlorodiisobutylaluminium (i-Bu₂AlCl), le triéthylaluminium (AlEt₃), le tripropylaluminium (Al(n-Pr)₃), le triisobutylaluminium (Al(i-Bu)₃), le diéthyl-éthoxyaluminium (Et₂AlOEt), le méthylaluminoxane (MAO), l'éthylaluminoxane et les méthylaluminoxanes modifiés (MMAO).

### L'additif

Optionnellement, le système catalytique comprend un ou plusieurs additifs.

Lorsque le système catalytique est à base de nickel, l'additif est choisi parmi,
- les composés de type azoté, tels que la triméthylamine, la triéthylamine, le pyrrole, le 2,5-diméthylyrrole, la pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-triflurométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline, la 1,10-phénanthroline, N-méthylpyrrole, N-butylpyrrole N-méthylimidazole, le N-butylimidazole, la 2,2'-bipyridine, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, ou
- les composés de type phosphine choisi indépendamment parmi la tributylphosphine, la triisopropylphosphine, la tricyclopentylphosphine, la tricyclohexylphosphine, la triphénylphosphine, la tris(o-tolyl)phosphine, le bis(diphénylphosphino)éthane, l'oxyde de trioctylphosphine, l'oxyde de triphénylphosphine, la triphénylphosphite, ou
- les composés répondant à la formule générale (I) ou un des tautomères dudit composé : 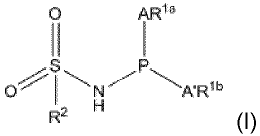 dans laquelle
   * A et A', identiques ou différents, sont indépendamment un oxygène ou une liaison simple entre l'atome de phosphore et un atome de carbone,
   * les groupements R^{1a} et R^{1b} sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 2-méthylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle,
   * le groupement R² est choisi indépendamment parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle.

Lorsque le système catalytique est à base de titane, l'additif est choisi parmi l'éther diéthylique, le diisopropyléther, le dibutyléther, le diphényléther, le 2-méthoxy-2-méthylpropane, 2-methoxy-2-méthylbutane, le diméthoxy-2,2 propane, le di(2-éthylhexyloxy)-2,2 propane, le 2,5-dihydrofurane, le tétrahydrofurane, le 2-méthoxytétrahydrofurane, le 2-méthyltétrahydrofurane, le 3-méthyltétrahydrofurane, le 2,3-dihydropyrane, le tétrahydropyrane, le 1,3-dioxolane, le 1,3-dioxane, le 1,4-dioxane, le diméthoxyéthane, di(2-méthoxyéthyl)éther, le benzofurane, le glyme et le diglyme pris seuls ou en mélange.

Lorsque le système catalytique est à base de chrome, l'additif est choisi parmi,
- les composés de type azoté, tels que la triméthylamine, la triéthylamine, le pyrrole, le 2,5-diméthylyrrole, la pyridine, la 2-méthylpyridine, la 3-méthylpyridine, la 4-méthylpyridine, la 2-méthoxypyridine, la 3-méthoxypyridine, la 4-méthoxypyridine, la 2-fluoropyridine, la 3-fluoropyridine, la 3-triflurométhylpyridine, la 2-phénylpyridine, la 3-phénylpyridine, la 2-benzylpyridine, la 3,5-diméthylpyridine, la 2,6-diterbutylpyridine et la 2,6-diphénylpyridine, la quinoline, la 1,10-phénanthroline, N-méthylpyrrole, N-butylpyrrole N-méthylimidazole, le N-butylimidazole, la 2,2'-bipyridine, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-diphényl-butane-2,3-diimine, la N,N'-bis-(diméthyl-2,6-phényl)-butane-2,3-diimine, la N,N'-bis-(diisopropyl-2,6-phényl)-butane-2,3-diimine, et/ou
- les composés aryloxy de formule générale [M(R³O)₂₋ₙXₙ]_{y} dans laquelle
   * M est choisi parmi le magnésium, le calcium, le strontium et le baryum, de préférence le magnésium,
   * R³ est un radical aryl contenant de 6 à 30 atomes de carbone, X est un halogène ou un radical alkyl contenant de 1 à 20 atomes de carbone,
   * n est un nombre entier qui peut prendre les valeurs de 0 ou 1, et
   * y est un nombre entier compris entre 1 et 10, de préférence y est égal à 1, 2, 3 ou 4.

De préférence, le radical aryloxy R³O est choisi parmi le 4-phénylphénoxy, le 2-phénylphénoxy, le 2,6-diphénylphénoxy, le 2,4,6-triphénylphénoxy, le 2,3,5,6-tétraphénylphénoxy, le 2-tert-butyl-6-phénylphénoxy, le 2,4-ditertbutyl-6-phénylphénoxy, le 2,6-diisopropylphénoxy, le 2,6-diméthylphénoxy, le 2,6-ditert-butylphénoxy, le 4-méthyl-2,6-ditert-butylphénoxy, le 2,6-dichloro-4-tert-butylphénoxy et le 2,6-dibromo-4-tert-butylphénoxy. Les deux radicaux aryloxy peuvent être portés par une même molécule, comme par exemple le radical biphénoxy, le binaphtoxy ou le 1,8-naphtalène-dioxy, De préférence, le radical aryloxy R³O est le 2,6-diphénylphénoxy, le 2-tert-butyl-6-phénylphénoxy ou le 2,4-ditert-butyl-6-phénylphénoxy.

### Le solvant

Dans un autre mode de réalisation selon l'invention, le système catalytique comprend optionnellement un ou plusieurs solvants.

Le solvant est choisi parmi le groupe formé par les hydrocarbures aliphatiques et cycloaliphatiques tels que l'hexane, le cyclohexane, l'heptane, le butane ou l'isobutane.

De manière préférée, le solvant utilisé est le cyclohexane.

Dans un mode de réalisation, un solvant ou un mélange de solvants peut être utilisé durant la réaction d'oligomérisation. Ledit solvant est avantageusement choisi indépendamment parmi le groupe formé par les hydrocarbures aliphatiques et cycloaliphatiques tels que l'hexane, le cyclohexane, l'heptane, le butane ou l'isobutane.

De préférence, les alpha oléfines linéaires obtenues comprennent de 4 à 20 atomes de carbone, de préférence de 4 à 18 atomes de carbones, de préférence de 4 à 10 atomes de carbones, et de préférence de 4 à 8 atomes de carbone. De manière préférée, les oléfines sont des alpha-oléfines linéaires, choisi parmi le but-1-ène, le hex-1-ène ou l'oct-1-ène.

Avantageusement, le procédé d'oligomérisation est mis en oeuvre à une pression comprise entre 0,1 et 10,0 MPa, de préférence entre 0,2 et 9,0 MPa et préférentiellement entre 0,3 et 8,0 MPa, à une température comprise entre 30 et 200°C, de préférence entre 35 et 150°C et de manière préférée entre 45 et 140°C.

De préférence, la concentration en catalyseur est comprise entre 0,01 et 500,0 ppm en masse de métal atomique par rapport à la masse réactionnelle, de préférence entre 0,05 et 100,0 ppm, de préférence entre 0,1 et 50,0 ppm et de préférence entre 0,2 et 30,0 ppm en masse de métal atomique par rapport à la masse réactionnelle.

Selon un autre mode de réalisation, le procédé d'oligomérisation est mise en oeuvre en continu. Le système catalytique, constitué comme décrit ci-dessus, est injecté en même temps que l'éthylène dans un réacteur agité par les moyens mécaniques classiques connus de l'homme du métier ou par une recirculation extérieure, et maintenu à la température souhaitée. On peut aussi injecter séparément les composants du système catalytique dans le milieu réactionnel. L'éthylène gazeux est introduit par une vanne d'admission asservie à la pression, qui maintient celle-ci constante dans le réacteur. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits issus de la réaction ainsi que le solvant sont séparés, par exemple par distillation. L'éthylène qui n'a pas été transformé peut être recyclé dans le réacteur. Les résidus de catalyseur inclus dans une fraction lourde peuvent être incinérés.

### Etape a) d'introduction du système catalytique

Le procédé mettant en oeuvre le réacteur à zones successives de diamètre variable selon l'invention comprend une étape a) d'introduction d'un système catalytique comprenant un catalyseur métallique et un agent activateur, et éventuellement d'un solvant ou d'un mélange de solvants, dans une enceinte réactionnelle comprenant une phase liquide dans une partie inférieure et un ciel gazeux dans une partie supérieure.

De préférence, l'introduction du système catalytique est réalisée dans la phase liquide dans la partie inférieure de l'enceinte réactionnelle et de préférence dans le fond de l'enceinte réactionnelle.

De préférence, la pression d'introduction dans l'enceinte réactionnelle est comprise entre 0,1 et 10,0 MPa, de préférence entre 0,2 et 9,0 MPa et préférentiellement entre 0,3 et 8,0 MPa.

De préférence la température d'introduction dans l'enceinte réactionnelle est entre 30 et 200°C, de préférence entre 35 et 150°C et de manière préférée entre 45 et 140°C.

### Etape b) de mise en contact avec de l'éthylène gazeux

Le procédé mettant en oeuvre le réacteur à zones de diamètre variable selon l'invention comprend une étape b) de mise en contact du système catalytique introduit à l'étape a) avec de l'éthylène gazeux. Ledit éthylène gazeux est introduit dans la phase liquide au niveau de la partie inférieure de l'enceinte réactionnelle, de préférence sur la partie inférieure latérale de l'enceinte réactionnelle. L'éthylène gazeux introduit comprend de l'éthylène gazeux frais, et de préférence, ledit éthylène gazeux frais est combiné avec de l'éthylène gazeux recyclé à une étape de séparation ultérieure au procédé d'oligomérisation.

Lors de la mise en oeuvre du procédé selon l'invention, suite à l'étape l'introduction de l'éthylène gazeux, la phase liquide comprend de l'éthylène gazeux non dissous, ainsi selon les zones de l'enceinte réactionnelle, la phase liquide correspond à un mélange gaz-liquide entre notamment la phase liquide et l'éthylène gazeux. De préférence, la zone dans le fond de l'enceinte réactionnelle sous le niveau d'introduction de l'éthylène gazeux comprend, de préférence est constituée, de la phase liquide sans éthylène gazeux.

De préférence, l'éthylène gazeux est distribué par dispersion lors de son introduction dans la phase liquide dans la partie inférieure de l'enceinte réactionnelle par un moyen apte à réaliser ladite dispersion de manière uniforme sur toute la section du réacteur. De préférence, le moyen de dispersion est choisi parmi un réseau distributeur avec une répartition homogène des points d'injection d'éthylène sur toute la section du réacteur.

De préférence, la vitesse de l'éthylène gazeux en sortie des orifices est comprise entre 1,0 et 30,0 m/s. Sa vitesse superficielle (vitesse volumique de gaz divisée par la section de l'enceinte réactionnelle) est comprise entre 0,5 et 10,0 cm/s et de préférence entre 1,0 et 8,0 cm/s.

De préférence, l'éthylène gazeux est introduit à un débit compris entre 1 et 250 t/h, de préférence entre 3 et 200 t/h, de préférence entre 5 et 150 t/h et de préférence entre 10 et 100 t/h.

De préférence, le débit d'éthylène gazeux introduit à l'étape b) est asservi à la pression dans l'enceinte réactionnelle.

Selon un mode particulier de mise en œuvre de l'invention, un flux d'hydrogène gazeux peut également être introduit dans l'enceinte réactionnelle, avec un débit représentant 0,2 à 1,0 % en masse du débit d'éthylène entrant. De préférence, le flux d'hydrogène gazeux est introduit par la conduite mise en œuvre pour l'introduction de l'éthylène gazeux.

### Etape c) de soutirage d'une fraction de la phase liquide

Le procédé mettant en œuvre le réacteur à zones de diamètre variable selon l'invention comprend une étape c) de soutirage d'une fraction de la phase liquide de préférence dans la partie inférieure de l'enceinte réactionnelle.

Le soutirage mis en œuvre à l'étape c) est, de préférence, réalisé dans la partie inférieure de l'enceinte réactionnelle, de préférence sous le niveau de l'injection d'éthylène gazeux, et de préférence dans le fond de l'enceinte. Le soutirage est mis en œuvre par tout moyen apte à réaliser le soutirage et de préférence par une pompe.

De préférence, le débit de soutirage est compris entre 500 et 10000 t/h, et de préférence entre 800 et 7000 t/h.

Dans un mode de réalisation, un second flux est soutiré de la phase liquide. Ledit second flux correspond à l'effluent obtenu à l'issue du procédé d'oligomérisation et peut être envoyé vers une section de séparation située en aval du dispositif mis en œuvre dans le procédé selon l'invention.

Selon un mode préféré de réalisation, la fraction liquide soutirée de la phase liquide est divisée en deux flux. Le premier flux dit principal est envoyé vers l'étape d) de refroidissement, et le second flux correspond à l'effluent et est envoyé vers la section aval de séparation.

Avantageusement, le débit dudit second flux est régulé pour maintenir un niveau liquide constant dans le réacteur. De préférence, le débit dudit second flux est de 5 à 200 fois inférieur au débit liquide envoyé à l'étape de refroidissement. De préférence, le débit dudit effluent est de 5 à 150 fois inférieur, de préférence de 10 à 120 fois inférieur et de manière préférée de 20 à 100 fois inférieur.

### Etape d) de refroidissement de la fraction liquide

Le procédé mettant en œuvre le réacteur à zones de diamètre variable selon l'invention comprend une étape d) de refroidissement de la fraction liquide soutirée à l'étape c).

De préférence, l'étape de refroidissement est mise en œuvre par la circulation du flux principal liquide soutiré à l'étape c), à travers un ou plusieurs échangeurs thermiques situés à l'intérieur ou à l'extérieur de l'enceinte réactionnelle et de préférence à l'extérieur.

L'échangeur thermique permet de diminuer la température de la fraction liquide de 1,0 à 30,0°C, de préférence entre 2,0 et 20°C, de préférence entre 2,0 et 15,0°C, de préférence entre 2,5 et 10,0°C, de préférence de 3,0 à 9,0°C, de préférence de 4,0 à 8,0°C. Avantageusement le refroidissement de la fraction liquide permet de maintenir la température du milieu réactionnel dans les gammes de température souhaitées.

Avantageusement, la mise en œuvre de l'étape de refroidissement du liquide, par l'intermédiaire de la boucle de recirculation permet également d'effectuer l'agitation du milieu réactionnel, et ainsi d'homogénéiser les concentrations des espèces réactives dans tout le volume liquide de l'enceinte réactionnelle.

### Etape e) d'introduction de la fraction liquide refroidie

Le procédé mettant en œuvre le réacteur à zones de diamètre variable selon l'invention comprend une étape e) d'introduction de la fraction liquide refroidie à l'étape d).

L'introduction de la fraction liquide refroidie issue de l'étape d) est réalisée dans la phase liquide de l'enceinte réactionnelle, de préférence dans la partie supérieure de ladite enceinte, par tout moyen connu de l'Homme du métier.

Avantageusement, lorsque la fraction refroidie est introduite dans la partie supérieure de la phase liquide contenue dans l'enceinte réactionnelle, un sens de circulation de ladite phase liquide est induite du sommet vers le fond de ladite enceinte ce qui ralentit la montée de l'éthylène gazeux dans la phase liquide et donc améliore la dissolution de l'éthylène dans la phase liquide. Ainsi la combinaison de ce mode de réalisation et du réacteur à zones de diamètre variable selon l'invention permet de limiter encore mieux le phénomène de perçage.

De préférence, le débit d'introduction de la fraction liquide refroidie est compris entre 500 et 10000 t/h, et de préférence entre 800 et 7000 t/h.

Les étapes c) à e) constituent une boucle de recirculation. Avantageusement, la boucle de recirculation permet d'effectuer l'agitation du milieu réactionnel, et ainsi homogénéiser les concentrations des espèces réactives dans tout le volume liquide de l'enceinte réactionnelle.

### Etape f) optionnelle de recyclage d'une fraction gazeuse soutirée du ciel gazeux

Le procédé mettant en oeuvre le réacteur à zones de diamètre variable selon l'invention comprend une étape f) de recyclage d'une fraction gazeuse soutirée du ciel gazeux de l'enceinte réactionnelle et introduite au niveau de la partie inférieure de l'enceinte réactionnelle dans la phase liquide, de préférence sur la partie inférieure latérale de l'enceinte réactionnelle, de préférence en fond de l'enceinte réactionnelle. La partie inférieure désigne le quart inférieur de l'enceinte réactionnelle.

L'étape f) de recyclage de la fraction gazeuse est encore appelée boucle de recycle. Le soutirage de la fraction gazeuse mis en oeuvre à l'étape f) est réalisé par tout moyen apte à réaliser le soutirage et de préférence par une pompe .

Un avantage de l'étape f) de recyclage est de permettre de compenser de façon simple et économique le phénomène de perçage de l'éthylène gazeux dans le ciel gazeux dans un procédé d'oligomérisation quelles que soient les dimensions du réacteur selon l'invention.

Le phénomène de perçage correspond à l'éthylène gazeux qui traverse la phase liquide sans se dissoudre et qui passe dans le ciel gazeux. Lorsque le débit d'éthylène gazeux injecté et le volume de ciel sont fixés à une valeur donnée, le perçage entraine alors une augmentation de pression dans l'enceinte réactionnelle. Dans un réacteur gaz/liquide mis en oeuvre selon un procédé préféré, le débit d'introduction de l'éthylène à l'étape b) est asservi à la pression dans l'enceinte réactionnelle. Ainsi, en cas d'augmentation de la pression dans le réacteur du fait d'un fort taux de perçage de l'éthylène dans le ciel gazeux, le débit d'éthylène gazeux introduit à l'étape b) diminue ce qui entraîne une diminution de la quantité d'éthylène dissous dans la phase liquide et donc de la saturation. La diminution de la saturation est préjudiciable pour la conversion de l'éthylène et s'accompagne d'une diminution de la productivité du réacteur. L'étape de recyclage d'une fraction gazeuse selon l'invention permet donc d'optimiser la saturation de l'éthylène dissous et donc d'améliorer la productivité volumique du procédé.

La fraction gazeuse soutirée à l'étape f) peut être introduite dans l'enceinte réactionnelle seule ou en mélange avec l'éthylène gazeux introduit à l'étape b). De préférence, la fraction gazeuse est introduite en mélange avec l'éthylène gazeux introduit à l'étape b).

Dans un mode de réalisation particulier, la fraction gazeuse soutirée à l'étape f) est introduite dans l'enceinte réactionnelle par dispersion dans la phase liquide dans la partie inférieure de l'enceinte réactionnelle par un moyen apte à réaliser ladite dispersion de manière uniforme sur toute la section du réacteur. De préférence, le moyen de dispersion est choisi parmi un réseau distributeur avec une répartition homogène des points d'injection de la fraction gazeuse soutirée à l'étape f) sur toute la section du réacteur.

De préférence, la vitesse de la fraction gazeuse soutirée en sortie des orifices est comprise entre 1,0 et 30,0 m/s. Sa vitesse superficielle (vitesse volumique de gaz divisée par la section de l'enceinte réactionnelle) est comprise entre 0,5 et 10,0 cm/s et de préférence entre 1,0 et 8,0 cm/s.

De préférence, le débit de soutirage de la fraction gazeuse est compris entre 0,1 et 100 % du débit d'éthylène gazeux introduit à l'étape b), de préférence 0,5 et 90,0 %, de préférence 1,0 et 80,0 %, de préférence entre 2,0 et 70,0 %, de préférence entre 4,0 et 60,0 %, de préférence entre 5,0 et 50,0 %, de préférence entre 10,0 et 40,0 % et de manière préférée entre 15,0 et 30,0 %.

Avantageusement, le débit de soutirage de la fraction gazeuse à l'étape f) est asservi à la pression au sein de l'enceinte réactionnelle ce qui permet de maintenir la pression à une valeur ou une gamme de valeurs souhaitée et donc de compenser le phénomène de perçage de l'éthylène gazeux dans le ciel.

Dans un mode de réalisation particulier, la fraction gazeuse soutirée à l'étape f) est divisée en deux flux, un premier flux gazeux dit principal est recyclé directement dans l'enceinte réactionnelle, et un second flux gazeux.

Dans un mode de réalisation préféré, ledit second flux gazeux correspond à une purge du ciel gazeux, qui permet d'éliminer une partie des gaz incondensables.

De préférence, le débit du second flux gazeux est compris entre 0,005 et 1,00 % du débit d'éthylène introduit à l'étape b), de préférence entre 0,01 et 0,50 %.

### EXEMPLES

Les exemples ci-dessous illustrent l'invention sans en limiter la portée.

### Exemple 1 : comparatif correspondant à la figure 1

L'exemple 1 met en oeuvre un réacteur gaz/liquide d'oligomérisation selon l'art antérieur, tel que décrit à la figure 1, comprenant une enceinte réactionnelle de forme cylindrique ayant un diamètre de 2,63 m et une hauteur de liquide de 4,31 m de hauteur liquide.

**Mise en oeuvre du procédé d'oligomérisation de l'éthylène selon l'art antérieur, à une pression de 7,0 MPa et à une température de 130°C comprenant les étapes suivantes** :
- on introduit le système catalytique à base de chrome, tel que décrit dans le brevet FR3019064 dans la phase liquide de l'enceinte réactionnelle en présence de cyclohexane comme solvant, avec un ratio du débit massique entrant de solvant sur le débit massique entrant d'éthylène de 1,
- on met en contact ledit système catalytique avec de l'éthylène gazeux en introduisant l'éthylène gazeux dans la partie inférieure de ladite enceinte,
- on récupère l'effluent réactionnel.

Les performances de ce réacteur permettent de convertir 79,6 % de l'éthylène gazeux injecté, et d'atteindre une sélectivité de 78,8 % en hexène-1. Ce réacteur permet l'obtention d'une saturation en éthylène dissous de 60,0 %, mesurée par analyse chromatographique en phase gaz d'un échantillon de la phase liquide soutirée de l'enceinte réactionnelle.

### Exemple 2 : selon l'invention correspondant à la figure 2

Un réacteur selon l'invention ayant deux zones de diamètre décroissant est mis en oeuvre dans les mêmes conditions que l'exemple 1.

Le tableau ci-dessous présente les résultats en saturation de l'éthylène dans la phase liquide pour quatre réacteurs ayant un volume total identique, mais dont les dimensions (en mètres) des deux zones selon l'invention sont différentes. La zone située en fond de l'enceinte réactionnelle est notée 1, la hauteur, le diamètre, et le volume correspondant sont respectivement notés H1, D1, et V1. La zone située au sommet de l'enceinte réactionnelle est notée 2, la hauteur, le diamètre, et le volume correspondant sont respectivement notés H2, D2, V2.

Le taux de saturation est mesuré par analyse chromatographique en phase gaz d'un échantillon de la phase liquide soutirée de l'enceinte réactionnelle.

| | Réacteur 1 | Réacteur 2 | Réacteur 3 | Réacteur 4 |
|---|---|---|---|---|
| Hauteur zone de fond (H1) | 3,017 | 3,017 | 1,293 | 1,293 |
| Diamètre zone de fond (D1) | 2,63 | 2,63 | 2,63 | 2,63 |
| Volume zone de fond (V1) | 16,39 | 16,39 | 7,02 | 7,02 |
| Hauteur zone au sommet (H2) | 2,02 | 5,17 | 4,71 | 12,07 |
| Diamètre zone au sommet (D2) | 2,02 | 1,315 | 2,104 | 1,315 |
| Volume zone au sommet (V2) | 7,02 | 7,02 | 16,39 | 16,39 |
| Volume global | 23,4 | 23,4 | 23,4 | 23,4 |
| Taux de saturation (%) | 77 | 95 | 84 | 97 |
| Conversion de l'éthylène (%) | 73,9 | 67,5 | 71,5 | 66,9 |
| Sélectivité hexène-1 (%) | 83,4 | 86,9 | 84,9 | 87,2 |

Les résultats présentés sont obtenus pour un ratio massique du débit de solvant injecté sur le débit d'éthylène gazeux injecté égal à 1.

Ces résultats illustrent clairement le gain de performances apporté par la mise en œuvre d'un réacteur selon l'invention. Ainsi un réacteur selon la présente invention permet d'obtenir une meilleure saturation en éthylène de la phase liquide, et donc une meilleure sélectivité en produit ciblé, ici le 1-hexène, pour un même volume total de réacteur, et pour un temps de séjour identique.

## Revendications

1. Réacteur d'oligomérisation gaz/liquide comprenant :
- une enceinte réactionnelle (1), de forme allongée le long d'un axe vertical,
- un moyen d'introduction d'éthylène gazeux (2), situé dans le fond de l'enceinte réactionnelle,
- un moyen de soutirage (5) d'un effluent liquide réactionnel situé dans le fond de l'enceinte réactionnelle,
- un moyen de purge (4) d'une fraction gazeuse situé au sommet dudit réacteur,
dans lequel
- ladite enceinte est composée de n zones consécutives ayant un diamètre Dn décroissant dans le sens de la zone de fond vers la zone du sommet de ladite enceinte,
- le rapport (Dn/Dn-1) du diamètre de la zone supérieure Dn, sur le diamètre de la zone inférieure adjacente Dn-1, est inférieur ou égale à 0,9,
- pour une zone donnée le rapport du volume Vn, sur le volume total de l'enceinte réactionnelle, Vtot, (Vn/Vtot) est compris entre 0,2 et 0,8.
- les n zones consécutives sont disposées en série selon l'axe vertical du réacteur de manière à définir des zones dans l'enceinte réactionnelle ayant des diamètres décroissants du fond vers le sommet et ainsi d'augmenter la hauteur d'une phase liquide pouvant être contenue dans ladite enceinte réactionnelle par rapport à la hauteur d'un réacteur de diamètre constant.

2. Réacteur selon la revendication 1 dans lequel le nombre n de zones est compris entre 2 et 5.

3. Réacteur selon l'une quelconque des revendications précédentes dans lequel le rapport (Dn/Dn-1) du diamètre d'une zone supérieure n sur le diamètre de la zone inférieure adjacente n-1 est compris entre 0,1 et 0,9.

4. Réacteur selon l'une quelconque des revendications précédentes dans lequel le rapport (Hn/Hn-1) de la hauteur d'une zone supérieure n, noté Hn, sur la hauteur de la zone inférieure adjacente n-1, noté Hn-1, est compris entre 0,2 et 3,0, de préférence entre 0,3 et 2,5.

5. Réacteur selon l'une quelconque des revendications précédentes dans lequel pour une zone donnée le rapport du volume noté Vn, sur le volume total, noté Vtot, (noté Vn/Vtot) de l'enceinte réactionnelle correspondant à la somme des n zones est compris entre 0,25 et 0,75.

6. Réacteur selon l'une quelconque des revendications 1 à 5 dans lequel les n zones composant ladite enceinte sont formées par des internes positionnés à l'intérieur de l'enceinte réactionnelle de manière à diminuer son diamètre sur une zone donnée.

7. Réacteur selon l'une quelconque des revendications précédentes comprenant en outre une boucle de recirculation comprenant un moyen de soutirage sur la partie inférieure de l'enceinte réactionnelle, de préférence au fond, de manière à soutirer une fraction liquide vers un ou plusieurs échangeur(s) thermique(s) apte(s) au refroidissement de ladite fraction liquide, et un moyen d'introduction de ladite fraction refroidie dans la partie supérieure de l'enceinte réactionnelle.

8. Réacteur selon l'une quelconque des revendications précédentes comprenant en outre un moyen de soutirage d'une fraction gazeuse au niveau du ciel gazeux de l'enceinte réactionnelle et un moyen d'introduction de ladite fraction gazeuse soutirée dans la phase liquide dans la partie inférieure de l'enceinte réactionnelle.

9. Procédé d'oligomérisation mettant en œuvre le réacteur selon l'une des revendications 1 à 8, ledit procédé étant mis en œuvre à une pression comprise entre 0,1 et 10,0 MPa, à une température comprise entre 30 et 200°C comprenant les étapes suivantes :
- une étape a) d'introduction d'un système catalytique d'oligomérisation comprenant un catalyseur métallique et un agent activateur, dans une enceinte réactionnelle,
- une étape b) de mise en contact dudit système catalytique avec de l'éthylène gazeux par l'introduction dudit éthylène gazeux dans la zone inférieure de l'enceinte réactionnelle,
- une étape c) de soutirage d'une fraction liquide,
- une étape d) de refroidissement de la fraction soutirée à l'étape c) par le passage de ladite fraction dans un échangeur thermique,
- une étape e) d'introduction de la fraction refroidie à l'étape d) dans la partie supérieure de la zone inférieure de l'enceinte réactionnelle.

10. Procédé selon la revendication 9 comprenant en outre une étape de recyclage d'une fraction gazeuse soutirée de la zone supérieure de l'enceinte réactionnelle et introduite au niveau de la partie inférieure de l'enceinte réactionnelle dans la phase liquide.

## Patentansprüche

1. Gas/Flüssig-Olimgomerisationsreaktor, umfassend:
- eine Reaktionskammer (1) von entlang einer vertikalen Achse länglicher Form,
- ein Einleitungsmittel (2) für gasförmiges Ethylen, das im Sumpf der Reaktionskammer gelegen ist,
- ein Abzugsmittel (5) für einen flüssigen Reaktionsabstrom, das im Sumpf der Reaktionskammer gelegen ist,
- ein Entlüftungsmittel (4) für eine gasförmige Fraktion, das am Kopf des Reaktors gelegen ist,
wobei
- die Kammer aus n aufeinander folgenden Zonen mit einem in Richtung von der Sumpfzone zur Kopfzone der Kammer hin abnehmenden Durchmesser Dn besteht,
- das Verhältnis (Dn/Dn-1) des Durchmessers der oberen Zone Dn zum Durchmesser der benachbarten unteren Zone Dn-1 kleiner oder gleich 0,9 ist,
- für eine gegebene Zone das Verhältnis des Volumens Vn zum Gesamtvolumen der Reaktionskammer, Vtot, (Vn/Vtot) zwischen 0,2 und 0,8 beträgt.
- die n aufeinander folgenden Zonen so in Reihe entlang der vertikalen Achse des Reaktors angeordnet sind, dass Zonen in der Reaktionskammer mit vom Sumpf zum Kopf hin abnehmendem Durchmesser definiert werden und so die Höhe einer flüssigen Phase, die in der Reaktionskammer enthalten sein kann, im Verhältnis zu der Höhe eines Reaktors mit konstantem Durchmesser vergrößert wird.

2. Reaktor nach Anspruch 1, wobei die Anzahl n der Zonen zwischen 2 und 5 beträgt.

3. Reaktor nach einem der vorhergehenden Ansprüche, wobei das Verhältnis (Dn/Dn-1) des Durchmessers einer oberen Zone n zum Durchmesser der benachbarten unteren Zone n-1 zwischen 0,1 und 0,9 beträgt.

4. Reaktor nach einem der vorhergehenden Ansprüche, wobei das Verhältnis (Hn/Hn-1) der Höhe einer oberen Zone n, bezeichnet als Hn, zur Höhe der benachbarten unteren Zone n-1, bezeichnet als Hn-1, zwischen 0,2 und 3,0, bevorzugt zwischen 0,3 und 2,5, beträgt.

5. Reaktor nach einem der vorhergehenden Ansprüche, wobei für eine gegebene Zone das Verhältnis des Volumens, bezeichnet als Vn, zum Gesamtvolumen, bezeichnet als Vtot, (bezeichnet als Vn/Vtot) der Reaktionskammer, das der Summe der n Zonen entspricht, zwischen 0,25 und 0,75 beträgt.

6. Reaktor nach einem der Ansprüche 1 bis 5, wobei die n Zonen, aus denen die Kammer besteht, durch Einbauten gebildet werden, die im Inneren der Reaktionskammer so positioniert sind, dass sie ihren Durchmesser über eine gegebene Zone verringern.

7. Reaktor nach einem der vorhergehenden Ansprüche, umfassend ferner einen Rezirkulationskreislauf, umfassend ein Abzugsmittel am unteren Teil der Reaktionskammer, bevorzugt am Sumpf, so dass eine flüssige Fraktion zu einem oder mehreren Wärmetauscher(n) abgezogen wird, der(die) zur Kühlung der flüssigen Fraktion geeignet ist (sind), und ein Einleitungsmittel zum Einleiten der gekühlten Fraktion in den oberen Teil der Reaktionskammer.

8. Reaktor nach einem der vorhergehenden Ansprüche, umfassend ferner ein Abzugsmittel zum Abziehen einer gasförmigen Fraktion an der Gasphase der Reaktionskammer und ein Einleitungsmittel zum Einleiten der abgezogenen gasförmigen Fraktion in die flüssige Phase in dem unteren Teil der Reaktionskammer.

9. Verfahren zur Oligomerisation unter Implementierung des Reaktors nach einem der Ansprüche 1 bis 8, wobei das Verfahren bei einem Druck zwischen 0,1 und 10,0 MPa, bei einer Temperatur zwischen 30 und 200 °C implementiert wird, umfassend die folgenden Schritte
- einen Schritt a) des Einleitens eines katalytischen Oligomerisationssystems, umfassend einen metallischen Katalysator und einen Aktivator, in eine Reaktionskammer,
- einen Schritt b) des Inkontaktbringens des katalytischen Systems mit gasförmigem Ethylen durch das Einleiten des gasförmigen Ethylens in die untere Zone der Reaktionskammer,
- einen Schritt c) des Abziehens einer flüssigen Fraktion,
- einen Schritt d) des Kühlens der im Schritt c) abgezogenen Fraktion durch das Durchlaufen der Fraktion durch einen Wärmetauscher,
- einen Schritt e) des Einleitens der im Schritt d) gekühlten Fraktion in den oberen Teil der unteren Zone der Reaktionskammer.

10. Verfahren nach Anspruch 9, umfassend ferner einen Schritt des Rückführens einer gasförmigen Fraktion, die aus der oberen Zone der Reaktionskammer abgezogen wird und am unteren Teil der Reaktionskammer in die flüssige Phase eingeleitet wird.

## Claims

1. Gas/liquid oligomerization reactor comprising:
- a reaction chamber (1), of elongate shape along a vertical axis,
- a means for introducing gaseous ethylene (2), located in the bottom of the reaction chamber,
- a means for withdrawing (5) a reaction liquid effluent, located in the bottom of the reaction chamber,
- a means for purging (4) a gaseous fraction, located at the top of said reactor;
in which
- said chamber is composed of n consecutive zones having a diameter Dn which decreases in the direction of the bottom zone to the top zone of said chamber,
- the ratio (Dn/Dn-1) of the diameter of the upper zone Dn, to the diameter of the adjacent lower zone Dn-1, is less than or equal to 0.9,
- for a given zone, the ratio of the volume Vn, to the total volume of the reaction chamber, Vtot, (Vn/Vtot) is between 0.2 and 0.8,
- the n consecutive zones are placed in series along the vertical axis of the reactor so as to define zones in the reaction chamber having diameters that decrease from the bottom to the top and thus to increase the height of a liquid phase that can be contained in said reaction chamber compared to the height of a constant-diameter reactor.

2. Reactor according to Claim 1, in which the number n of zones is between 2 and 5.

3. Reactor according to either one of the preceding claims, in which the ratio (Dn/Dn-1) of the diameter of an upper zone n to the diameter of the adjacent lower zone n-1 is between 0.1 and 0.9.

4. Reactor according to any one of the preceding claims, in which the ratio (Hn/Hn-1) of the height of an upper zone n, denoted Hn, to the height of the adjacent lower zone n-1, denoted Hn-1, is between 0.2 and 3.0, preferably between 0.3 and 2.5.

5. Reactor according to any one of the preceding claims, in which, for a given zone, the ratio of the volume, denoted Vn, to the total volume, denoted Vtot (said ratio being denoted Vn/Vtot), of the reaction chamber corresponding to the sum of the n zones is between 0.25 and 0.75.

6. Reactor according to any one of Claims 1 to 5, in which the n zones making up said chamber are formed by internals positioned inside the reaction chamber so as to reduce its diameter over a given zone.

7. Reactor according to any one of the preceding claims, also comprising a recirculation loop comprising a withdrawal means on the lower part of the reaction chamber, preferably at the bottom, so as to withdraw a liquid fraction to one or more heat exchanger(s) capable of cooling said liquid fraction, and a means for introducing said cooled fraction into the upper part of the reaction chamber.

8. Reactor according to any one of the preceding claims, also comprising a means for withdrawing a gaseous fraction at the level of the gaseous headspace of the reaction chamber and a means for introducing said withdrawn gaseous fraction into the liquid phase in the lower part of the reaction chamber.

9. Oligomerization process using the reactor according to one of Claims 1 to 8, said process being carried out at a pressure between 0.1 and 10.0 MPa, at a temperature between 30°C and 200°C, comprising the following steps:
- a step a) of introducing a catalytic oligomerization system comprising a metal catalyst and an activating agent into a reaction chamber,
- a step b) of bringing said catalytic system into contact with gaseous ethylene by introducing said gaseous ethylene into the lower zone of the reaction chamber,
- a step c) of withdrawing a liquid fraction,
- a step d) of cooling the fraction withdrawn in step c) by passing said fraction through a heat exchanger,
- a step e) of introducing the fraction cooled in step d) into the upper part of the lower zone of the reaction chamber.

10. Process according to Claim 9, also comprising a step of recycling a gaseous fraction, withdrawn from the upper zone of the reaction chamber and introduced at the lower part of the reaction chamber, into the liquid phase.
